# EUROPÄISCHE PATENTSCHRIFT

(11) **EP 3 695 763 B1**
(45) Veröffentlichungstag und Bekanntmachung des Hinweises auf die Patenterteilung: **09.03.2022**
(21) Anmeldenummer: 20153184.5
(22) Anmeldetag: 22.01.2020
(51) Int. Cl.: A47J 47/02

(54) **MESSVORRICHTUNG FÜR EINEN DECKEL ZUM ABDECKEN EINES BEHÄLTERS**
MEASURING DEVICE FOR A LID FOR COVERING A CONTAINER
DISPOSITIF DE MESURE POUR UN COUVERCLE DESTINÉ À RECOUVRIR UN RÉCIPIENT

(30) Priorität: 12.02.2019 DE 102019201771
(43) Veröffentlichungstag der Anmeldung: 19.08.2020
(73) Patentinhaber: BSH Hausgeräte GmbH, 81739 München (DE)
(72) Erfinder: Buck, Thorbjörn, 82229 Türkenfeld (DE); Bui Tran, Duc Hanh, 81735 München (DE); Söllner, Christoph, 81475 München (DE)

(56) Entgegenhaltungen:
- CN-A- 107 960 833
- DE-A1-102016 200 762
- US-A1- 2017 172 352

## Beschreibung

Die Erfindung betrifft eine Messvorrichtung für einen Deckel zum Verschließen eines Behälters, insbesondere um in energieeffizienter Weise Information in Bezug auf den Inhalt des Behälters bereitzustellen.

In einem Haushalt werden Nahrungsmittel in zahlreichen unterschiedlichen Behältern aufbewahrt. Dabei ist es für einen Nutzer typischerweise schwierig einen Überblick über die in den unterschiedlichen Behältern gelagerten Substanzen (z.B. Nahrungsmittel oder Reinigungsflüssigkeiten oder Reinigungspulver) zu behalten. Insbesondere kann es vorkommen, dass ein Nutzer ein bestimmtes Gericht nicht herstellen kann, weil er fälschlicherweise angenommen hatte, dass in einem bestimmten Behälter noch eine ausreichende Menge eines bestimmten Nahrungsmittels zur Herstellung des bestimmten Gerichtes vorrätig sei.

Aus der CN 107 960 833 A ist ein Kochgerät mit einer Kamera vorbekannt, die in einem Vorratsbehälter eine Menge einer verbleibenden Substanz misst. Die DE 10 2016 200 762 A1 beschäftigt sich mit einer Kaffeemaschine mit einem kapazitiven Füllstandssensor. Aus der US 2017/0172352 A1 ist ein Behälter mit einem Verschlusssensor vorbekannt, der in seinem Inneren ein Vakuum erzeugen kann.

Das vorliegende Dokument befasst sich mit der technischen Aufgabe, in effizienter Weise präzise Information über den Inhalt von ein oder mehreren Behältern (in einem Haushalt) bereitzustellen und/oder zu nutzen, insbesondere, um den Komfort für einen Nutzer der ein oder mehreren Behälter zu erhöhen.

Die Aufgabe wird jeweils durch den Gegenstand der unabhängigen Patentansprüche gelöst. Vorteilhafte Ausführungsformen sind insbesondere in den abhängigen Patentansprüchen definiert, in nachfolgender Beschreibung beschrieben oder in der beigefügten Zeichnung dargestellt.

Gemäß einem Aspekt der Erfindung wird eine Messvorrichtung für einen Deckel zum Abdecken eines Behälters beschrieben, wobei der Behälter zur Aufbewahrung einer Substanz verwendet wird. Die Messvorrichtung umfasst zumindest einen Vorrichtungssensor, der eingerichtet ist, Vorrichtungsinformation in Bezug auf eine Bewegung der Messvorrichtung relativ zu dem Behälter bereitzustellen, sowie zumindest einen Inhaltssensor, der eingerichtet ist, Substanz-Sensordaten in Bezug auf die Substanz in dem Behälter zu erfassen, sowie zumindest eine Kommunikationseinheit, die eingerichtet ist, Daten über ein Kommunikationsnetzwerk zu versenden. Der Vorrichtungssensor umfasst einen Bewegungssensor und die Vorrichtungsinformation Bewegungsdaten des Bewegungssensors.

Außerdem umfasst die Messvorrichtung eine Steuereinheit, die eingerichtet ist, auf Basis der Bewegungsdaten zu bestimmen, ob der mit der Messvorrichtung verbundene Deckel auf den Behälter aufgesetzt wurde oder von dem Behälter abgenommen wurde, und in Abhängigkeit von der Vorrichtungsinformation den Inhaltssensor zu veranlassen, Substanz-Sensordaten zu erfassen. Des Weiteren ist die Steuereinheit eingerichtet, die Kommunikationseinheit zu veranlassen, die Substanz-Sensordaten und/oder davon abgeleitete Daten zu versenden.

Gemäß einem weiteren Aspekt der Erfindung wird ein Verfahren zur Bereitstellung von Information in Bezug auf eine Substanz beschrieben, die in einem Behälter aufbewahrt wird. Das Verfahren kann mittels einer Messvorrichtung für einen Deckel zum Abdecken des Behälters ausgeführt werden. Das Verfahren umfasst das Ermitteln von Vorrichtungsinformation in Bezug auf eine Bewegung der Messvorrichtung relativ zu dem Behälter, wobei die Vorrichtungsinformation Bewegungsdaten des Bewegungssensors umfasst. Gemäß dem Verfahren wird auf Basis der Bewegungsdaten bestimmt, ob der mit der Messvorrichtung verbundene Deckel auf den Behälter aufgesetzt wurde oder von dem Behälter abgenommen wurde. Des Weiteren umfasst das Verfahren das Erfassen von Substanz-Sensordaten mittels der Messvorrichtung, in Abhängigkeit von der Vorrichtungsinformation. Außerdem umfasst das Verfahren das Versenden der Substanz-Sensordaten und/oder davon abgeleiteter Daten.

Es ist zu beachten, dass jegliche Aspekte des in diesem Dokument beschriebenen Systems, des in diesem Dokument beschriebenen Verfahrens und/oder der in diesem Dokument beschriebenen Vorrichtung in vielfältiger Weise miteinander kombiniert werden können. Insbesondere können die Merkmale der Patentansprüche in vielfältiger Weise miteinander kombiniert werden.

Im Weiteren wird die Erfindung anhand von in der beigefügten Zeichnung dargestellten Ausführungsbeispielen näher beschrieben. Dabei zeigen
- Figur 1a: einen bespielhaften Deckel für einen Behälter, wobei der Deckel eine Messvorrichtung umfasst;
- Figur 1b: ein beispielhaftes System zur Verwaltung einer Vielzahl von Behältern;
- Figuren 2a: bis 2c beispielhafte Ereignis-Diagramme für den Betrieb einer Messvorrichtung;
- Figuren 3a: und 3b eine beispielhafte Messvorrichtung, die an einem Deckel befestigt werden kann; und
- Figur 4: ein Ablaufdiagramm eines beispielhaften Verfahrens zum Betrieb einer Messvorrichtung.

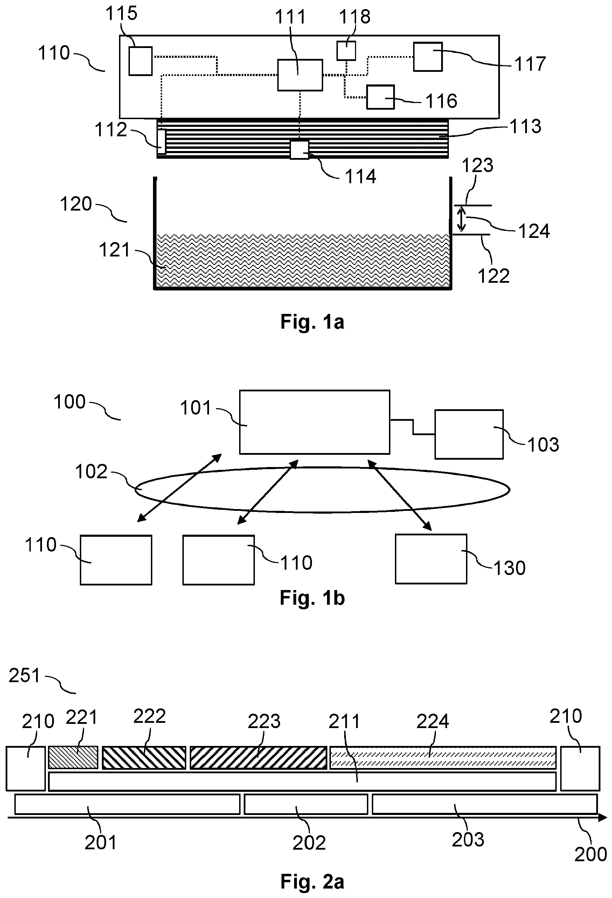

Wie eingangs dargelegt, befasst sich das vorliegende Dokument mit der effizienten Bereitstellung und Nutzung von Information in Bezug auf den Inhalt zumindest eines Behältnisses bzw. Behälters. In diesem Zusammenhang zeigt Fig. 1a einen beispielhaften Deckel 110, der eingerichtet ist, ein Behältnis (d.h. einen Behälter) 120 abzudecken. Insbesondere kann der Deckel 110 ein Gewinde 113 aufweisen, mit dem der Deckel 110 in ein entsprechendes Gewinde (nicht dargestellt) des Behältnisses 120 eingeschraubt werden kann, um das Behältnis 120 mit dem Gewinde 113 zu verschließen.

In dem Behältnis 120 kann sich eine Substanz 121 (insbesondere eine flüssige oder pulverförmige Substanz) befinden. Die Menge der Substanz 121, die sich in dem Behältnis 120 befindet, kann z.B. durch den Füllstand 122 der Substanz 121 in dem Behältnis 120 angezeigt werden. Beispielhafte Substanzen 121 sind (essbare) Pulver oder Flüssigkeiten.

Der Deckel 110 umfasst zumindest einen Inhaltssensor 114, der eingerichtet ist, Sensordaten in Bezug auf den Inhalt, insbesondere in Bezug auf die Substanz 121, in einem Behältnis 120 zu erfassen. Der Inhaltssensor 114 kann insbesondere einen Abstandssensor umfassen, der eingerichtet ist, den Abstand 124 zwischen der Position 123 des Inhaltssensors 114 (im eingeschraubten Zustand) und dem Füllstand 112 der in dem Behältnis 120 enthaltenen Substanz 121 zu messen.

Die (Substanz-) Sensordaten des zumindest einen Inhaltssensors 114 können von einer Steuereinheit 111 des Deckels 110 verarbeitet werden (z.B. um auf Basis der Substanz-Sensordaten den Füllstand 122 zu ermitteln). Alternativ oder ergänzend können die Sensordaten und/oder davon abgeleitete Daten über eine Kommunikationseinheit 117 des Deckels 110 versendet werden (z.B. an eine Zentral- bzw. Backendeinheit). Die Kommunikationseinheit 117 kann eingerichtet sein, die Daten über eine drahtgebundene (z.B. LAN) und/oder über ein drahtlose (z.B. WLAN, Bluetooth und/oder Zigbee) Kommunikationsverbindung zu versenden. Des Weiteren kann der Deckel 110 eine Speichereinheit 116 zur Speicherung von Daten und/oder einen (ggf. wiederaufladbaren) elektrischen Energiespeicher bzw. Batterie 115 zur elektrischen Energieversorgung der Komponenten 111, 112, 114, 116, 117, 118 des Deckels 110 aufweisen.

Der Deckel 110 weist einen Verschlusssensor 112 (in diesem Dokument auch allgemein als Vorrichtungssensor bezeichnet) auf, der eingerichtet ist, zu detektieren, ob der Deckel 110 ein Behältnis 120 verschließt oder nicht. Insbesondere kann der Verschlusssensor 112 eingerichtet sein, zu detektieren, ob der Deckel 110 (ggf. vollständig) auf ein Behältnis 120 aufgeschraubt wurde oder nicht. Der Verschlusssensor 112 kann z.B. einen Taster umfassen, der mechanisch durch eine Wand des Behältnisses 120 betätigt wird, wenn der Deckel 110 auf das Behältnis 120 aufgebracht wird.

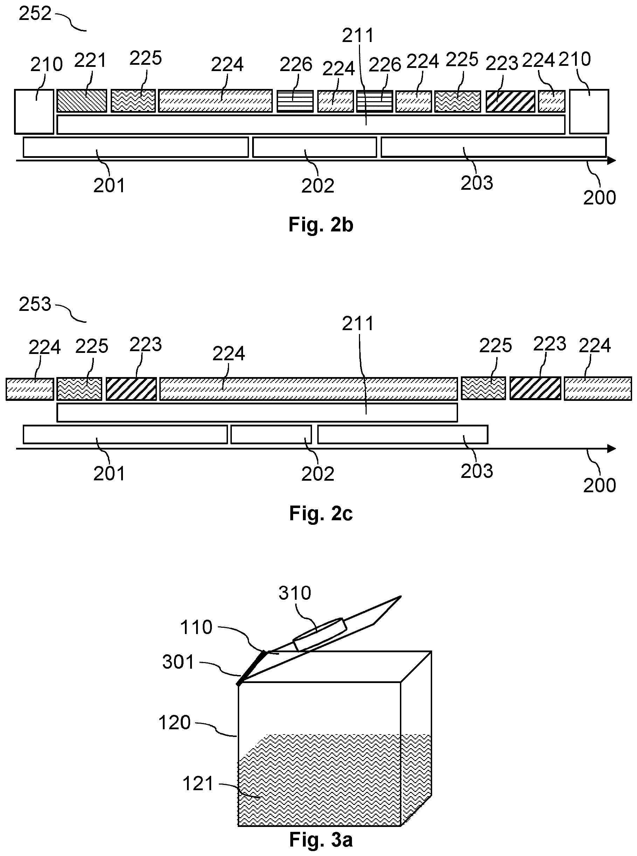

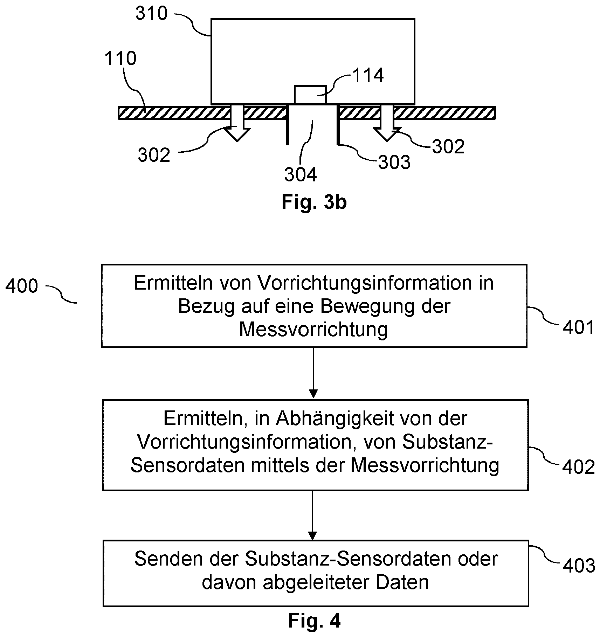

Fig. 1a zeigt einen Deckel 110, der bestimmte Komponenten 111, 112, 114, 115, 116, 117, 118 zur Erfassung und/oder zur Bereitstellung von Substanz-Sensordaten in Bezug auf die Substanz 121 in einem Behälter 120 umfasst. Wie beispielhaft in Zusammenhang mit den Figuren 3a und 3b dargelegt, können die Komponenten 111, 112, 114, 115, 116, 117, 118 des Deckels 110 in einer separaten Messvorrichtung 310 verbaut sein, wobei die Messvorrichtung 310 mit einem Deckel 110 verbindbar ist. Die in diesem Dokument beschriebenen Aspekte sind sowohl für einen Deckel 110 mit einer integrierten Messvorrichtung 310 (wie z.B. in Fig. 1a dargestellt) als auch für einen Deckel 110 mit einer separaten Messvorrichtung 310 (wie z.B. in Fig. 3a dargestellt) anwendbar.

Fig. 1b zeigt ein beispielhaftes System 100 zur Verwaltung des Inhalts von ein oder mehreren Behältnissen 120. Das System 100 umfasst ein oder mehrere Deckel 110 zur Abdeckung von entsprechenden ein oder mehreren Behältnissen 120. Die einzelnen Deckel 110 (bzw. daran befestigte Messvorrichtungen 310) können eingerichtet sein, über ein (drahtloses) Kommunikationsnetz 102 mit einer Zentraleinheit 101 (z.B. mit einem Server) zu kommunizieren, insbesondere um Daten in Bezug auf den Inhalt der ein oder mehreren Behältnisse 120 bereitzustellen.

Die Zentraleinheit 101 kann eingerichtet sein, mit ein oder mehreren persönlichen elektronischen Geräten 130 (z.B. einem Smartphone, einem Tablet PC, einem Laptop PC, etc.) eines Nutzers des Systems 100 über ein (drahtloses) Kommunikationsnetz 102 Daten auszutauschen. Insbesondere können einem Nutzer auf dem Gerät 130 Daten in Bezug auf den Inhalt der ein oder mehreren Behältnisse 120 bereitgestellt werden.

Die Zentraleinheit 101 kann Zugriff auf eine Datenbank 103 haben, um die Güte der ermittelten und/oder bereitgestellten Information zu dem Inhalt der ein oder mehreren Behältnisse 120 zu erhöhen. Die Datenbank 103 kann z.B. Information in Bezug auf das Fassungsvolumen der ein oder mehreren Behältnisse 120 und/oder Information in Bezug auf die Dichte von unterschiedlichen Substanzen 121 umfassen.

Es wird somit ein System 100 beschrieben, das zumindest einen (intelligenten) Deckel 110 umfasst, der z.B. mit einem Gewinde 113 auf einen beliebigen (Flüssigkeits)-Behälter 120 aufgeschraubt werden kann, beispielsweise auf eine 2L-Flasche mit Flüssigwaschmittel. Am unteren Ende des Deckels 110 kann ein Abstandssensor 114 angeordnet sein (der z.B. auf einer Ultraschallmessung, einer Laser-Ranging Messung, einer Reflexionsmessung und/oder auf einer Laufzeitmessung beruht). Der Abstandssensor 114 ist bevorzugt derart angebracht, dass der Abstandssensor 114 mit seinem Erfassungsbereich die Oberfläche einer in einem Behälter 120 enthaltenen Substanz 121 (z.B. einer Flüssigkeit oder eines Pulvers) erfassen kann. Dabei kann der Sensor 114 in unterschiedlichen Ausführungsformen gestaltet sein (z.B. Time-of-Flight, Ultraschall, Widerstandsstab mit unterschiedlichen Segmenten, Schwimmer mit Triggerpunkt, etc.), um (Substanz-) Sensordaten in Bezug auf den Abstand 124 bzw. den Füllstand 122 bereitzustellen.

Alternativ oder ergänzend kann das Behältnis 120 selbst Teil des Messsystems sein. Beispielsweise können zwei an gegenüberliegenden Seiten des Behälters 120 aufgebrachte Leiter zur Füllstandsdetektion eine variable Kapazität liefern. In diesem Fall enthält die Auswerteelektronik 114 im Deckel 110 entsprechende Komponenten zur Erfassung der füllstandsabhängigen Kapazität.

Der Deckel 110 kann auf einem Gefäß 120 durch Schrauben oder Stecken (Haftreibung) fixiert werden. Dabei kann in dem Deckel 110 ein Sensor 112, z.B. ein Taster, derart angebracht sein, dass der Sensor 112 ein durch einen Nutzer begonnenes Entfernen des Deckels 110 von einem Behälter 120 detektieren kann. Alternativ oder ergänzend zu einem Taster kann eine Lichtschranke und/oder eine Reflexionslichtschranke verwendet werden, um das Entfernen des Deckels 110 von einem Behälter 120 zu detektieren. Dabei kann der Sensor 112 eingerichtet sein, in horizontaler und/oder in vertikaler Richtung einen Kontakt zwischen dem Deckel 110 und dem Behälter 120 zu detektieren.

Der Deckel 110 weist eine Steuereinheit 111 auf, die mit den Sensoren 112, 114 verbunden ist. Der Deckel 110 kann einen lokalen dynamischen und/oder persistenten Datenspeicher 116 umfassen. Des Weiteren kann der Deckel 110 über eine Kommunikationseinheit 117 und über ein Kommunikationsnetz 102 mit einer Zentraleinheit 101 zum Datenaustausch verbunden sein.

Die Zentraleinheit 101 kann Zugriff auf Information darüber haben, welche Formen und/oder Volumina unterschiedliche Typen von Behältern 120 aufweisen. Diese Information kann z.B. auf einer Datenbank 103 gespeichert sein. Damit kann die Zentraleinheit 101 aus einem von einem Deckel 110 (insbesondere von einem Inhaltssensor 114) erfassten Messwert und auf Basis von Information über die verwendete Produktart ("Waschmittel") einen tatsächlichen Füllstand 122 und/oder eine tatsächliche Füllmenge der Substanz 121 in einem Behälter 120 ermitteln, auch wenn der Behälter 120 eine nicht-zylindrische Form aufweist.

Des Weiteren kann die Zentraleinheit 101 über Verbindungen zu verschiedenen weiteren Systemen, z.B. einer Home-Connect-Applikation und/oder einer generischen Schnittstelle, aufweisen, z.B. um Information in Bezug auf den Füllstand 122 und/oder verarbeitete Information, wie z.B. einen Trigger bzw. einen Hinweis für nicht ausreichend verfügbare Substanz 121 in einem Behälter 120, bereitzustellen. Alternativ oder ergänzend können durch die Zentraleinheit 101 ein oder mehrere Dienstleistungen bereitgestellt werden, wie z.B. das automatische Nachbestellen einer Substanz 121, sobald erkannt wird, dass der Füllstand 122 in einem Behälter 120 zu niedrig ist.

Die Figuren 2a bis 2c beschreiben unterschiedliche Verfahren zur Bereitstellung von Inhaltsdaten (bzw. Substanz-Sensordaten) in Bezug auf den Inhalt eines Behälters 120 durch einen Deckel 110. Fig. 2a zeigt ein beispielhaftes Verfahren 251, bei dem durch einen Nutzer der Deckel 110 von einem (ggf. leeren) Behälter 120 abgeschraubt wird (Schritt 201), der Deckel 110 anschließend für eine bestimmte Zeit vom Behälter 120 entfernt wird (Schritt 202), z.B. neben den Behälter 120 gelegt wird, und dann der Deckel 110 wieder auf den Behälter 120 aufgesetzt wird (Schritt 203). Die Tatsache, dass der Deckel 110 von dem Behälter 120 entfernt wird oder auf den Behälter 120 aufgesetzt wird, kann durch den Verschlusssensor 112 detektiert werden (Schritt 211). Vor dem Zeitpunkt, an dem ein Entfernen des Deckels 110 detektiert wird, und nach dem Zeitpunkt, an dem ein Wiederaufsetzten des Deckels 110 detektiert wird, kann sich der Deckel 110 in einem deaktivierten und/oder Aus-Zustand 210 befinden. Der Deckel 110 kann z.B. ausgebildet sein, in dem Aus-Zustand 210 einen äußerst geringen oder gar keinen Energieverbrauch (z.B. von (nahezu) Null) aufzuweisen.

Nach dem Detektieren des Entfernens des Deckels 110 kann der Deckel 110 gestartet bzw. gebootet werden (Schritt 221), und es kann daraufhin durch den zumindest einen Inhaltssensor 114 ein Messvorgang durchgeführt werden (Schritt 222). Die erfassten (Substanz-) Sensordaten und/oder davon abgeleitete Daten können dann über die Kommunikationseinheit 117 versendet werden (Schritt 223). Anschließend kann der Deckel 110 in einem Schlaf- bzw. Deep Sleep-Zustand 224 übergehen, in dem der Deckel 110 einen reduzierten und/oder minimalen Energieverbrauch (z.B. von nahezu 0mA) aufweist.

Der Verschlusssensor 112 (insbesondere der Taster) kann derart gestaltet und montiert sein, dass der Verschlusssensor 112 mit Zuschrauben des Deckels 112 auf ein Gefäß 120 eingedrückt wird. Dabei kann eine Kontaktierung derart erfolgen, dass der Verschlusssensor 112 bereits bei einer relativ kleinen öffnenden Umdrehung vom vollständig zugeschraubten Zustand, z.B. bei einer öffnenden Umdrehung von 90°, auslöst. Solange der Deckel 110 vollständig zugeschraubt ist, kann sich der Deckel 110 (bevorzugt stromlos) in einem Ruhe- oder Aus-Zustand 210 befinden. Mit geringfügigem Öffnen des Deckels 110 kann der Deckel 110 (z.B. innerhalb von Millisekunden) gestartet werden (Schritt 221) und kann anschließend mindestens eine Abstandsmessung eines Abstandssensors 114 durchführen (z.B. in Form einer "Burstmessung"). Die Messung kann dabei erfolgen während der Nutzer den Deckel 110 weiter aufdreht. Die dadurch zunehmende Entfernung von der Oberfläche der Substanz 121 liegt jedoch auch bei einem relativ steilen Gewinde 113 im Millimeterbereich, so dass ein dadurch bewirkter Messfehler typischerweise vernachlässigbar ist.

Sobald eine Messung abgeschlossen ist, kann der Deckel 110 den Messwert mit Hilfe der Kommunikationseinheit 117 über eine geeignete Kommunikationsarchitektur an die Zentraleinheit 101 senden (Schritt 223). Anschließend kann der Deckel 110 in einen Schlaf-Zustand 224 versetzt werden (in dem der Deckel 110 annähernd stromlos ist). Sobald der Nutzer den Deckel 110 wieder fest auf das Behältnis 120 aufschraubt, wird der Verschlusssensor 112 wieder betätigt und kann dabei den Deckel 110, z.B. durch Unterbrechung der Stromversorgung, vollständig stromlos schalten (und somit in den Ruhe-Zustand 210 versetzen). Eine daran anschließende Messung erfolgt in entsprechender Weise.

Fig. 2b beschreibt ein alternatives Verfahren 252, bei dem wiederholt (relativ genaue) Burst-Messungen (Schritt 225) und/oder (relativ ungenaue) Einfach-Messungen (Schritt 226) durchgeführt werden, während der Deckel 110 abgenommen und/oder aufgesetzt wird. Zwischen den einzelnen Messungen kann der Deckel 110 in einen energiesparenden Schlaf-Zustand 224 versetzt werden.

Wenn ein Behälter 120 (z.B. eine Waschmittelpackung) leer ist, kann der Nutzer den Deckel 110 von dem leeren Behälter 120 entfernen, und auf einen neuen und/oder befüllten Behälter 120 aufsetzen. Durch das in Fig. 2a beschriebene Verfahren 251 kann der Füllstand 122 des befüllten Behälters 120 typischerweise nicht erfasst werden, da die Messung beim Abnehmen des Deckels 110 erfolgt. Eine Messung des neuen Füllstands 122 erfolgt erst nach erneutem Abnehmen des Deckels 110.

In dem in Fig. 2b dargestellten Verfahren 252 wird nachdem der Deckel 110 leicht abgeschraubt wurde, eine Burstmessung 225 durchgeführt (wie auch im Verfahren 251). Allerdings geht der Deckel 110 danach nicht dauerhaft in den Schlaf-Zustand 224 über, sondern führt weiterhin in bestimmten Zeitintervallen, z.B. alle 5 Sekunden, singuläre Abstandsmessungen 226 durch. Wenn diese Messung in einem definierten Wertebereich liegt, wobei der Wertebereich typische Größen von Behältern 120 berücksichtigt, kann eine erneute Burstmessung 225 durchgeführt werden. Es können so lange Burstmessungen 225 durchgeführt werden, bis sich die Messungen nicht mehr wesentlich voneinander unterscheiden. Es kann dann davon ausgegangen werden, dass der Deckel 110 wieder auf einen Behälter 110 aufgesetzt wurde. Der so ermittelte Messwert kann dann an die Zentraleinheit 101 gesendet werden (Schritt 223), und der Deckel 110 kann in den Schlaf-Zustand 224 und anschließend in den Ruhe-Zustand 210 übergehen (wie bereits in Zusammenhang mit dem Verfahren 251 beschrieben).

Das Verfahren 252 verbraucht typischerweise mehr Energie als das Verfahren 251, weil wiederholt Messungen durchgeführt werden. Andererseits kann durch das Verfahren 252 in präziser Weise der Füllstand 122 eines Behälters 120 beim Aufsetzten des Deckels 110 ermittelt werden (z.B. zu Beginn eines Zuschraubvorgangs). Der Deckel 110 kann ggf. stromlos geschaltet werden, sobald der Zuschraubvorgang vollständig beendet ist.

Fig. 2c zeigt ein Verfahren 253, bei dem durch ein von dem Verschlusssensor 112 detektiertes Verschließ- und/oder Öffnungs-Ereignis, ein Trigger bewirkt wird, der eine Messung (insbesondere eine Bustmessung 225) veranlasst. So können direkt beim Abnehmen des Deckels 110 und/oder beim Aufsetzten des Deckels 110 Messdaten bereitgestellt werden. Zwischen den Messungen 225 kann sich der Deckel 110 in einem Schlaf-Zustand 224 befinden. Der Deckel 110 kann somit eingerichtet sein, Level-Trigger zu generieren, indem der Verschlusssensor 112 direkt mit einem Eingang der Steuereinheit 111 gekoppelt ist. Der Deckel 110 kann dabei kontinuierlich mit Strom versorgt werden, z.B. um den Deckel 110 im Schlaf-Zustand 224 zu betreiben. Erst wenn der Taster entweder losgelassen ("Aufschrauben") oder gedrückt ("Zuschrauben") wird, startet eine Burst-Messung 225 mit anschließendem Senden 223 von Messdaten an die Zentraleinheit 101. Das Verfahren 253 kann in energieeffizienter Weise durch ein "System-on-Chip, SoC" Design implementiert werden. Des Weiteren ermöglicht das Verfahren 253 eine zeitlich flexible Kommunikation der Messdaten an die Zentraleinheit 101, da der Deckel 110 typischerweise nicht in den Ruhe-Zustand 210 überführt wird. So kann vermieden werden, dass Messwerte nicht an die Zentraleinheit 101 übertragen werden können und dabei verlorengehen.

Die Kommunikationseinheit 117 kann eingerichtet sein, ein oder mehrere drahtlose Kommunikationstechnologien und/oder Standards zu ermöglichen. Die zu übertragenden Daten können z.B. den gemessenen Füllstand 122 und/oder einen Messwert des Abstands 124 vom Sensor 114, sowie eine eineindeutige Identifikation, z.B. eine Seriennummer, des Deckels 110 bzw. der Messvorrichtung 310 umfassen.

Beispielhafte Kommunikationstechnologien sind:
- WLAN mit IEEE 802.11: Mit beendeter Messung 222, 225 kann die Steuereinheit 101 den WiFi-Chip aufwecken, und das Einbuchen in ein lokales WLAN-Netz anstoßen. Nach erfolgreichem Einbuchen können die Daten (ggf. verschlüsselt) an die Zentraleinheit 101 gesendet werden. Im Falle von WLAN wird eine dedizierte Verbindung aufgebaut, so dass eine sichere Datenübertragung ermöglicht wird.
- Bluetooth Legacy: Hierbei wird eine authentifizierte Verbindung zu einem geeigneten Gateway verwendet. Mit beendeter Messung 222, 225 baut die Kommunikationseinheit 117 die authentifizierte Verbindung zu dem Gateway (der sich z.B. in einem Hausgerät befinden kann) auf, und überträgt die Daten. Die Zwischenspeicherung der Daten und die Umsetzung auf ein Internetprotokoll(-IP)-basiertes Netz kann in dem Gateway erfolgen. Vorteilhaft ist, dass keine eigene Sicherheitslösung in der Kommunikationseinheit implementiert und betrieben werden muss.
- Bluetooth Low Energy (BLE): Die Kommunikationseinheit 117 sendet, sobald eine Messung beendet wurde, den Messwert zumindest signiert ohne Verbindungsaufbau an ein BLE-Gateway (der z.B. durch ein Hausgerät bereitgestellt wird). Die Sicherheit der Datenübertragung kann durch ein oder mehrere Maßnahmen zur Verhinderung von Angriffen (z.B. Manipulation, Replay, etc.) bewirkt werden.
- ZigBee: funktioniert analog zu BLE.
- Proprietär.

Die in diesem Dokument beschriebene Vorrichtung 310 zur Erfassung von Inhaltsdaten in Bezug auf den Inhalt, insbesondere in Bezug auf die Substanz 121, in einem Behälter 120 kann ggf. separat von einem Deckel 110 zur Abdeckung eines Behälters 120 bereitgestellt werden. Dies ist beispielhaft in den Figuren 3a und 3b dargestellt. Insbesondere zeigt Fig. 3a einen quaderförmigen Behälter 120 mit einem Deckel 110 (z.B. einen Behälter 120 für Waschmittel). Der Deckel 110 kann z.B. um eine Achse 301 geschwenkt werden, um den Deckel 110 zu öffnen, und um sich Zugang zu dem Innenraum des Behälters 120 zu verschaffen (z.B. um eine Substanz 121 aus dem Behälter 120 zu entnehmen).

An dem Deckel 110 des Behälters 120 ist eine Messvorrichtung 310 angeordnet, die die in Zusammenhang mit dem Deckel 110 aus Fig. 1a dargestellten Komponenten umfassen kann, um Inhaltsdaten (insbesondere Substanz-Sensordaten) in Bezug auf den Inhalt des Behälters 120 zu erfassen und bereitzustellen. Die Messvorrichtung 310 kann z.B. über einen Klettverschluss und/oder Haltenasen 302 (siehe Fig. 3b) mit dem Deckel 110 verbindbar sein. Beispielsweise kann die Messvorrichtung 310 derart ausgebildet sein, dass die Messvorrichtung 310 lösbar mit dem Deckel 110 verbunden werden kann. Dabei kann die Messvorrichtung 310 über einer Öffnung 304 im Deckel 110 positionierbar sein.

Alternativ oder ergänzend kann die Messvorrichtung 310 einen rohrförmigen Stempel 303 aufweisen, mit dem die Öffnung 304 in einem Deckel 110 erzeugt werden kann, wenn die Messvorrichtung 310 auf den Deckel 110 gedrückt wird, um die Messvorrichtung 310 an dem Deckel 110 zu befestigen. Die Öffnung 304 kann an der Stelle des Inhaltssensors 114 der Messvorrichtung 310 angeordnet sein, so dass der Inhaltssensor 114 durch die Öffnung 304 hindurch (Substanz-) Sensordaten in Bezug auf die Substanz 121 in dem Behälter 120 erfassen kann.

Wenn der Behälter 120 leer ist, kann die Messvorrichtung 310 von dem Deckel 110 getrennt werden, um die Messvorrichtung 310 an dem Deckel 110 eines anderen Behälters 120 befestigen zu können.

Es wird somit eine Messvorrichtung 310 beschrieben, die ein Gehäuse mit einer geeigneten Form, z.B. quaderförmig, aufweisen kann. Die Messvorrichtung 310 ist ausgebildet, auf den beweglichen Deckel 110 eines Vorratsbehälters 120 mechanisch abschließend angebracht zu werden. Die lösbare Verbindung zwischen der Messvorrichtung 310 und einem Deckel 110 kann auf unterschiedliche Art und Weise erfolgen, z.B. über einen Klettverschluss, und/oder über beliebig geformte Haltenasen 302, die in vorgesehene Bohrungen in einem Deckel 110 geschoben werden können und die auf der Innenseite des Deckels 110 durch herausklappende Elemente eine kraftschlüssige Verbindung herstellen können.

Typischerweise weist eine pulverförmige Substanz 121 (wie z.B. Waschmittel) in der Mitte eines Vorratsbehälters 120 die niedrigste Füllhöhe 122 auf, da ein Nutzer meist in der Mitte mit einem Schöpflöffel das Pulver entnimmt. Es ist daher vorteilhaft, die Messvorrichtung 310 möglichst mittig im Deckel anzubringen. Des Weiteren kann sich im Deckel 110 eine Öffnung 304 befinden (oder erzeugt werden), durch die ein Inhaltssensor 114 eine Abstandsmessung zur Ermittlung des Füllstands 122 durchführen kann.

Der Deckel 110 kann, wie Fig. 3a dargestellt, klappbar an dem Behälter 120 angebracht sein und kann z.B. zur Entnahme des Inhalts um bis zu 270° neben den Behälter 120 umklappbar sein. Die an den Deckel 110 angebrachte Messvorrichtung 310 kann dabei mitbewegt werden. Die Messvorrichtung 310 kann einen Bewegungssensor 118 umfassen (z.B. einen Beschleunigungssensor oder eine vollständige Inertial-Measurement-Unit (IMU)), der eingerichtet ist, ein Umklappen des Deckels 110 zu detektieren. Des Weiteren kann der Bewegungssensor 118 eingerichtet sein, ein oder mehrere bestimmte Bewegungs- und/oder Erschütterungsmuster zu detektieren. Die Steuereinheit 111 der Messvorrichtung 310 kann eingerichtet sein, das Erfassen und/oder Versenden von Inhaltsdaten (insbesondere von Substanz-Sensordaten) in Bezug auf den Inhalt eines Behälters 120 in Abhängigkeit von den Sensordaten des Bewegungssensors 118 zu steuern. Die Inhaltsdaten können an eine Zentraleinheit 101 gesendet werden, um die Inhaltsdaten auszuwerten und/oder um Dienstleistungen bereitzustellen (wie z.B. das automatische Nachbestellen von vollen Behältern 120 und/oder von neuen Substanzen 121).

Wie bereits oben dargelegt, ist die Messvorrichtung 310 bevorzugt für eine Steckmontage ausgelegt, um ein einfaches Wechseln der Messvorrichtung 310 von dem Deckel 110 eines Behälters 120 auf einen Deckel 110 eines anderen Behälters 120 (insbesondere eines anderen Verpackungskartons) zu ermöglichen. Dabei sollte die (Steck-) Verbindung sicher halten, um auch einer Bewegung des Deckels 110 folgen zu können, ohne dass sich die Messvorrichtung 310 von dem Deckel 110 löst. Dazu können beispielsweise Haltenasen 302 verwendet werden, die über klappbare Fixierungselemente verfügen. Während des Durchsteckens werden die klappbaren Fixierungselemente der Haltenasen 302 eingedrückt und schnappen nach beendetem Einsteckvorgang wieder aus dem Durchführungsstift der Haltenase 302 heraus, um die Messvorrichtung 310 an dem Deckel 110 zu fixieren. Zum Lösen der Verbindung kann der (z.B. aus Pappe bestehende) Deckel 110 zerstört werden. Alternativ kann eine Mechanik an der Messvorrichtung 310 verbaut sein, die z.B. in Reaktion auf ein mechanisches Einwirken (Knopfdruck, Zusammendrücken, etc.) die oben beschriebenen Fixierungselemente der Haltenasen 302 weit genug einzieht, damit die Messvorrichtung 310 von dem Deckel 110 gelöst werden kann.

In einer Ruhestellung befindet sich der Deckel 110 des Behälters 120 typischerweise in einer definierten Position, z.B. waagrecht zur Schwerkraft, auf dem Vorratsbehälter 120. Die Messvorrichtung 310 befindet sich dabei meist für relativ lange Zeit in einer bestimmten Ruhelage. Die Steuereinheit 111 und/oder der Bewegungssensor 118 können eingerichtet sein, zu detektieren, dass sich die Messvorrichtung 310 in einer Ruhelage befindet. Die Messvorrichtung 310 kann daraufhin in einem Schlaf-Zustand 224 betrieben werden.

Sobald zur Entnahme von einer Substanz 121 aus dem Behälter 120 der Deckel 110 aufgeklappt oder entfernt wird, kann von der Steuereinheit 111 und/oder von dem Bewegungssensor 118 eine Lageänderung und/oder eine Beschleunigung detektiert werden. Nach erfolgter Entnahme wird der Deckel 110 typischerweise wieder auf das Behältnis 120 und/oder in die Ruhelage gebracht. Die Steuereinheit 1110 und/oder der Beschleunigungssensor 118 können dabei eine Rückkehr in die Ruhelage detektieren, z.B. auf Basis des Beschleunigungsanteils, der wieder den betraglich gleichen Vektor wie beim Aufklappen annimmt und/oder auf Basis einer ausbleibenden Drehrate eines Drehratensensors 118.

Die Steuereinheit 111 kann dann eingerichtet sein (wenn erkannt wird, dass der Deckel 110 wieder geschlossen wurde), eine Messung in Bezug auf den Inhalt, insbesondere in Bezug auf den Füllstand 122, zu veranlassen. Zu diesem Zweck kann der Schlaf-Zustand 224 verlassen werden. Die erfassten Inhaltsdaten (z.B. die Substanz-Sensordaten) können durch die Kommunikationseinheit 117 an eine Zentraleinheit 101 gesendet werden. Die Messung kann ggf. nach Ablauf einer bestimmten Ruhezeit erfolgen, um der Substanz 121 im Anschluss an die Entnahme Zeit zum Ausbalancieren zu geben. So kann die Güte der erfassten Inhaltdaten bzw. Substanz-Sensordaten erhöht werden.

Die gemessenen Informationen des Bewegungssensors 118 können verwendet werden, um einem Nutzer ein potentielles Fehlverhalten anzuzeigen. Beispielsweise kann erkannt werden, dass der Nutzer den Deckel 110 nicht wieder richtig verschlossen hat (z.B., dass der Deckel 110 nicht in einer waagrechten Lage ist). Es kann dann ein Hinweis an den Nutzer veranlasst werden (z.B. durch Senden einer Nachricht an ein persönliches elektronisches Gerät 130 des Nutzers), um den Nutzer auf das Fehlverhalten hinzuweisen. Die Warnung und/oder der Hinweis können solange bestehen bleiben, bis der Fehlerzustand behoben ist. So kann die Aufbewahrungsgüte einer Substanz 121 erhöht werden. Beispielsweise können das Risiko des vorzeitigen Verklebens und der Verlust der Rieseleigenschaft eines Waschmittels reduziert werden.

Fig. 4 zeigt ein Ablaufdiagramm eines beispielhaften Verfahrens 400 zur Bereitstellung von Information in Bezug auf eine (flüssige oder pulverförmige) Substanz 121, die in einem Behälter 120 aufbewahrt wird. Die Information kann dabei mittels einer Messvorrichtung 310 für einen Deckel 110 zum Abdecken des Behälters 120 bereitgestellt werden. Dabei kann die Messvorrichtung 310 zumindest einen Vorrichtungssensor 112, 118 umfassen, der eingerichtet ist, Vorrichtungsinformation in Bezug auf eine Bewegung der Messvorrichtung 310 relativ zu dem Behälter 120 bereitzustellen. Des Weiteren kann die Messvorrichtung 310 zumindest einen Inhaltssensor 114 umfassen, der eingerichtet ist, Substanz-Sensordaten in Bezug auf die Substanz 121 (insbesondere in Bezug auf den Füllstand 122 der Substanz 121) in dem Behälter 120 zu erfassen. Außerdem kann die Messvorrichtung 310 zumindest eine Kommunikationseinheit 117 umfassen, die eingerichtet ist, Daten über ein Kommunikationsnetzwerk 102 zu versenden (z.B. an eine Zentraleinheit 101).

Das Verfahren 400 umfasst das Ermitteln 401 von Vorrichtungsinformation in Bezug auf eine Bewegung der Messvorrichtung 310 relativ zu dem Behälter 120. Die Vorrichtungsinformation kann mittels des Vorrichtungssensors 112, 118 der Messvorrichtung 310 ermittelt werden. Des Weiteren umfasst das Verfahren 400 das Erfassen 402 von Substanz-Sensordaten in Abhängigkeit von der Vorrichtungsinformation. Dabei kann insbesondere der Zeitpunkt des Erfassens von Substanz-Sensordaten in Abhängigkeit von der Vorrichtungsinformation ermittelt bzw. ausgewählt werden. Insbesondere kann ggf. nur dann ein Erfassen der Substanz-Sensordaten erfolgen, wenn die Vorrichtungsinformation anzeigt, dass der mit der Messvorrichtung verbundene Deckel 110 zumindest teilweise den Behälter 120 abdeckt. Durch die Berücksichtigung von Vorrichtungsinformation können in zuverlässiger Weise präzise Substanz-Sensordaten erfasst werden.

Das Verfahren 400 umfasst ferner das Versenden 403 der Substanz-Sensordaten und/oder davon abgeleiteter Daten. Die Daten können z.B. an eine Zentraleinheit 101 gesendet werden, und dazu genutzt werden, um einen Nutzer des Behälters 120 zu informieren und/oder um automatisch eine Nachbestellung von Substanz 121 (z.B. zum Auffüllen des Behälters 120) zu initiieren. So kann der Komfort für einen Nutzer bei der Lagerung von ein oder mehreren Substanzen 121 erhöht werden.

Es wird somit in diesem Dokument eine Messvorrichtung 310 für einen Deckel 110 zum Abdecken eines Behälters 120 beschrieben. Der Behälter 120 kann zur Aufbewahrung einer Substanz 121 verwendet werden. Beispielhafte Substanzen 121 sind Pulver oder Flüssigkeiten (z.B. Waschpulver oder Nahrungsmittel). Die Messvorrichtung 310 kann ausgebildet sein, um an einem Deckel 110 zum Abdecken eines Behälters 120 lösbar befestigt zu werden. In diesem Fall kann die Messvorrichtung 310 ein Gehäuse aufweisen, das Komponenten der Messvorrichtung 310 umschließt. Alternativ kann die Messvorrichtung 310 als fester Bestandteil in einen Deckel 110 integriert sein. Das die Komponenten der Messvorrichtung 310 umschließende Gehäuse kann dann durch den Deckel 110 gebildet werden.

Die Messvorrichtung 310 umfasst zumindest einen Vorrichtungssensor 112, 118, der eingerichtet ist, Vorrichtungsinformation in Bezug auf eine Bewegung der Messvorrichtung 310 relativ zu dem Behälter 120 bereitzustellen. Insbesondere kann die Vorrichtungsinformation anzeigen, ob ein mit der Messvorrichtung 310 verbundener Deckel 110 den Behälter 120 abdeckt oder nicht. Alternativ oder ergänzend kann die Vorrichtungsinformation anzeigen, ob ein mit der Messvorrichtung 310 verbundener Deckel 110 von dem Behälter 120 entfernt oder auf den Behälter 120 gesetzt wird.

Der Vorrichtungssensor 112, 118 kann z.B. einen Verschlusssensor 112 umfassen, der eingerichtet ist, zu detektieren, ob der mit der Messvorrichtung 310 verbundene Deckel 110 von dem Behälter 120 entfernt oder auf den Behälter 120 aufgesetzt wird. Der Verschlusssensor 112 kann beispielsweise einen Taster und/oder eine Lichtschranke umfassen. Dabei kann der Verschlusssensor 112 eingerichtet sein, in horizontaler und/oder in vertikaler Richtung einen Kontakt zwischen dem Deckel 110 und dem Behälter 120 zu detektieren.

Die Vorrichtungsinformation kann z.B. in binärer Weise anzeigen, ob der Kontakt des Verschlusssensors 112 geschlossen ist oder geöffnet ist. Der binäre Zustand des Verschlusssensors 112 kann dann anzeigen, ob der Deckel 110 von dem Behälter 120 entfernt oder auf den Behälter 120 aufgesetzt wird. Mittels eines Verschlusssensors 112 kann in effizienter Weise detektiert werden, dass ein Deckel 110 (mit der Messvorrichtung 310) relativ zu einem Behälter 120 bewegt wird, und dass daher davon ausgegangen werden kann, dass der Deckel 110 von dem Behälter 120 entfernt oder auf den Behälter aufgesetzt wird.

Alternativ oder ergänzend kann der Vorrichtungssensor 112, 118 einen Bewegungssensor 118, insbesondere einen Beschleunigungssensor und/oder eine Inertial Measurement Unit, umfassen. Die Vorrichtungsinformation kann dann Bewegungsdaten des Bewegungssensors 118 umfassen. Die Bewegungsdaten können analysiert werden, um Bewegungsmuster zu erkennen. Insbesondere kann ein Bewegungsmuster erkannt werden, das das Abnehmen eines Deckels 110 von dem Behälter 120 anzeigt. Des Weiteren kann ein Bewegungsmuster erkannt werden, das anzeigt, dass ein Deckel 110 auf einen Behälter 120 aufgesetzt wird. Es kann somit in präziser Weise auf Basis der Bewegungsdaten bestimmt werden, ob ein mit der Messvorrichtung 310 verbundener Deckel 110 auf einen Behälter 120 aufgesetzt wurde oder von einem Behälter 120 abgenommen wurde.

Des Weiteren umfasst die Messvorrichtung 310 zumindest einen Inhaltssensor 114, der eingerichtet ist, Substanz-Sensordaten in Bezug auf die Substanz 121 in dem Behälter 120 zu erfassen. Der Inhaltssensor 114 kann z.B. einen Abstandssensor umfassen, der eingerichtet ist, Substanz-Sensordaten in Bezug auf den Abstand 124 zwischen dem Abstandssensor und der Oberfläche der Substanz 121 in dem Behälter 120 zu erfassen. Auf Basis der Substanz-Sensordaten kann dann in präziser Weise der Füllstand 122 der Substanz 121 in dem Behälter 120 ermittelt werden.

Auf Basis der Vorrichtungsinformation kann insbesondere ein Zeitpunkt für die Messung der Substanz-Sensordaten bestimmt werden. Insbesondere kann eine Messung der Substanz-Sensordaten (ggf. nur dann) erfolgen, wenn die Vorrichtungsinformation anzeigt, dass sich der mit der Messvorrichtung 310 verbundene Deckel 110 (noch oder wieder) auf dem Behälter 120 befindet. So kann eine präzise Messung von Substanz-Sensordaten ermöglicht werden. Des Weiteren kann so der Energieverbrauch der Messvorrichtung reduziert werden, da Messungen der Substanz-Sensordaten unterbunden werden können, wenn sich der Deckel 110 nicht auf dem Behälter 120 befindet.

Außerdem umfasst die Messvorrichtung 310 zumindest eine Kommunikationseinheit 117, die eingerichtet ist, Daten über ein (drahtloses) Kommunikationsnetzwerk 102 zu versenden. Beispielhafte Kommunikationstechnologien sind WLAN, Bluetooth, Zigbee, etc. Die Kommunikationseinheit 117 kann dabei eingerichtet sein (ggf. indirekt über ein Gateway), Daten an eine Zentraleinheit 101 (z.B. an einen Backend-Server) zu senden.

Die Messvorrichtung 310 umfasst ferner eine Steuereinheit 111 (z.B. mit ein oder mehreren Mikroprozessoren), die eingerichtet ist, in Abhängigkeit von der Vorrichtungsinformation den Inhaltssensor 114 zu veranlassen, Substanz-Sensordaten zu erfassen. Insbesondere kann in Abhängigkeit von der Vorrichtungsinformation ein Zeitpunkt für die Erfassung von Substanz-Sensordaten ermittelt werden, um in energieeffizienter Weise präzise Substanz-Sensordaten erfassen zu können. Die Steuereinheit 111 kann außerdem eingerichtet sein, die Kommunikationseinheit 117 zu veranlassen, die Substanz-Sensordaten und/oder davon abgeleitete Daten zu versenden (z.B. an eine Zentraleinheit 101).

Es wird somit eine Messvorrichtung 310 beschrieben, die mit einem Deckel 110 verbunden und/oder Teil eines Deckels 110 sein kann, und die es einem Nutzer ermöglicht, in energieeffizienter Weise präzise Information in Bezug auf den Inhalt von ein oder mehreren Behältern 120 zu ermitteln.

Die Messvorrichtung 310 kann einen elektrischen Energiespeicher 115 (z.B. eine (ggf. wiederaufladbare) Batterie) umfassen, der eingerichtet ist, elektrische Energie für den Betrieb der Messvorrichtung 310 zu speichern. Durch die Bereitstellung eines elektrischen Energiespeichers 115 (der z.B. einen Betrieb für 6 Monate oder mehr, bzw. für 1 Jahr oder mehr ermöglicht) wird ein autarker Betrieb der Messvorrichtung 310 ermöglicht.

Alternativ oder ergänzend kann die Messvorrichtung 310 eine Speichereinheit 116 umfassen, die eingerichtet ist, zumindest vorübergehend Substanz-Sensordaten zu speichern. Durch die Zwischenspeicherung von Substanz-Sensordaten kann ein zeitlich flexibles Senden der Substanz-Sensordaten oder davon abgeleiteter Daten ermöglicht werden (z.B. um einen Datenverlust zu vermeiden).

Der Vorrichtungssensor 112, 118 (insbesondere der Verschlusssensor 112) kann eingerichtet sein, als Vorrichtungsinformation die Energieversorgung der Steuereinheit 111 zu unterbinden, wenn der mit der Messvorrichtung 310 verbundene Deckel 110 von dem Behälter 120 entfernt wird oder ist, oder wenn der mit der Messvorrichtung 310 verbundene Deckel 110 auf den Behälter 120 aufgesetzt wird oder ist. Alternativ oder ergänzend kann der Vorrichtungssensor 112, 118 (insbesondere der Verschlusssensor 112) eingerichtet sein, als Vorrichtungsinformation die Energieversorgung der Steuereinheit 111 nur dann zu ermöglichen, wenn der mit der Messvorrichtung 310 verbundene Deckel 110 von dem Behälter 120 entfernt wird oder ist, oder wenn der mit der Messvorrichtung 310 verbundene Deckel 110 auf den Behälter 120 aufgesetzt wird oder ist.

Der Vorrichtungssensor 112, 118 kann somit eingerichtet sein, die Vorrichtungsinformation in binärer Weise als "Energieversorgung an" bzw. "Energieversorgung aus" bereitzustellen. So kann ein besonders energieeffizienter Betrieb der Messvorrichtung 310 ermöglicht werden, da die Messvorrichtung 310 zumindest zeitweise deaktiviert sein kann (z.B., wenn der mit der Messvorrichtung 310 verbundene Deckel 110 von dem Behälter 120 entfernt wird bzw. wurde).

Die Steuereinheit 111 kann eingerichtet sein, auf Basis der Vorrichtungsinformation zu bestimmen, dass der mit der Messvorrichtung 310 verbundene Deckel 110 von dem Behälter 120 entfernt wird oder ist, oder dass der mit der Messvorrichtung 310 verbundene Deckel 110 auf den Behälter 120 aufgesetzt wird oder ist. Es kann dann, in Reaktion darauf, der Inhaltssensor 114 veranlasst werden, Substanz-Sensordaten zu erfassen, und die Kommunikationseinheit 117 kann veranlasst werden, die Substanz-Sensordaten und/oder davon abgeleitete Daten zu versenden.

Im Anschluss an das Erfassen und/oder Versenden von Substanz-Sensordaten kann die Messvorrichtung 310 (zumindest zeitweise) in einen Schlaf-Zustand 224 mit reduziertem Energieverbrauch überführt werden. Dabei kann der Schlaf-Zustand 224 derart ausgelegt sein, dass die Messvorrichtung sich wiederholt bzw. periodisch selbständig aus dem Schlaf-Zustand 224 wecken kann, z.B. um eine Messung von Substanz-Sensordaten vorzunehmen. Durch den zumindest vorübergehenden Übergang in einen Schlaf-Zustand 224 kann der Energieverbrauch der Messvorrichtung 310 reduziert werden.

Die Steuereinheit 111 kann eingerichtet sein, den Inhaltssensor 114 zu veranlassen, erste Substanz-Sensordaten mit einer reduzierten Genauigkeit in einem Energiespar-Modus (z.B. gemäß einer Single-Messung) zu erfassen. Es kann dann auf Basis der ersten Substanz-Sensordaten bestimmt werden, ob der mit der Messvorrichtung 310 verbundene Deckel 110 auf dem Behälter 120 aufgesetzt ist oder nicht. Insbesondere können die ersten Substanz-Sensordaten auf ihre Plausibilität hin überprüft werden. Die ersten Substanz-Sensordaten können wiederholt, insbesondere periodisch, erfasst werden.

Der Inhaltssensor 114 kann veranlasst werden, Substanz-Sensordaten mit einer (gegenüber der reduzierten Genauigkeit erhöhten) Standard-Genauigkeit in einem Standard-Modus zu erfassen, (ggf. nur dann) wenn oder sobald bestimmt wird, dass der mit der Messvorrichtung 310 verbundene Deckel 110 auf dem Behälter 120 aufgesetzt ist. Die Messvorrichtung 310 kann somit eingerichtet sein, zunächst in energieeffizienter Weise erste Substanz-Sensordaten mit einer relativ geringen Genauigkeit zu messen, die dann dazu verwendet werden können, in zuverlässiger Weise zu ermitteln, ob der Deckel 110 auf dem Behälter 120 liegt oder nicht. Die definitiven Substanz-Sensordaten können dann mit einem erhöhten Energieaufwand ermittelt werden, sobald sichergestellt wurde, dass der Deckel 110 tatsächlich auf dem Behälter 120 aufliegt. Durch das Erfassen von Substanz-Sensordaten mit unterschiedlicher Genauigkeit kann der Energieverbrauch der Messvorrichtung 310 weiter reduziert werden.

Die Messvorrichtung 310 kann ein oder mehrere Haltenasen 302 umfassen, die ausgebildet sind, durch einen Deckel 110 hindurch gesteckt zu werden, um die Messvorrichtung 310 an dem Deckel 110 zu befestigen. Alternativ oder ergänzend kann die Messvorrichtung 310 an einem Gehäuse ein Verbindungsmittel (z.B. ein Klettband oder einen Magneten) umfassen, das ausgebildet ist, mit einem komplementären Verbindungsmittel (z.B. mit einem komplementären Klettband oder einem Metallstück) an einem Deckel 110 eine wiederholt lösbare Verbindung (z.B. eine Klettverbindung oder eine magnetische Verbindung) zu bilden, um die Messvorrichtung 310 an dem Deckel 110 zu befestigen. Die Messvorrichtung 310 kann somit Mittel umfassen, die es einem Nutzer ermöglichen, die Messvorrichtung 310 in stabiler und wieder lösbarer Weise mit einem Deckel 110 zu verbinden. Die Messvorrichtung 310 kann somit in flexibler Weise für unterschiedliche Deckel 110 verwendet werden.

Die Messvorrichtung 310 kann ausgebildet sein, von außen an einem Deckel 110 befestigt zu werden. Des Weiteren kann der Inhaltssensor 114 der Messvorrichtung 310 ausgebildet sein, die Substanz-Sensordaten durch eine Öffnung 304 in dem Deckel 110 zu erfassen. Dabei kann die Messvorrichtung 310 ein rohrförmiges Stanzwerkzeug 303 umfassen, das ausgebildet ist, eine Öffnung 304 in dem Deckel 110 für den Inhaltssensor 114 auszustanzen, wenn die Messvorrichtung 310 an dem Deckel 110 befestigt wird. So wird eine flexible Befestigung der Messvorrichtung 310 an einem Deckel 110 ermöglicht.

Des Weiteren wird in diesem Dokument ein Deckel 110 zum Abdecken eines (Haushalts-) Behälters 120 beschrieben, wobei der Deckel 110 eine in diesem Dokument beschriebene Messvorrichtung 310 umfasst.

Der Deckel 110 kann ein Gewinde 113 umfassen, das ausgebildet ist, in ein entsprechendes Gewinde eines (Haushalts-) Behälters 120 geschraubt zu werden. Der Vorrichtungssensor 112, 118 (insbesondere der Verschlusssensor 112) der Messvorrichtung 310 kann derart in oder an dem Gewinde 113 des Deckels 110 angeordnet sein, dass die Vorrichtungsinformation anzeigt, ob der Deckel 110 von dem Behälter 120 abgeschraubt oder auf den Behälter 120 aufgeschraubt wird. Durch die Anordnung eines Vorrichtungssensors 112, 118 an dem Gewinde 113 eines Deckels 110 kann die Vorrichtungsinformation in besonders zuverlässiger Weise ermittelt werden.

Des Weiteren wird in diesem Dokument ein System 100 zur Verwaltung des Inhalts von ein oder mehreren (Haushalts-) Behältern 120 beschrieben. Das System 100 umfasst ein oder mehrere in diesem Dokument beschriebene Messvorrichtungen 110, wobei die ein oder mehrere Messvorrichtungen 110 ausgebildet sind, mit ein oder mehreren Deckeln 110 zum Abdecken der ein oder mehreren (Haushalts-) Behälter 120 verbunden zu werden, und/oder wobei die ein oder mehrere Messvorrichtungen 110 mit ein oder mehreren Deckeln 110 zum Abdecken der ein oder mehreren (Haushalts-) Behälter 120 verbunden sind.

Außerdem umfasst das System 100 eine Zentraleinheit 101, die eingerichtet ist, Substanz-Sensordaten und/oder davon abgeleitete Daten in Bezug auf den Inhalt der ein oder mehreren Behälter 120 von den ein oder mehreren Messvorrichtungen 310 zu empfangen. Die Zentraleinheit 101 ist ferner eingerichtet, in Abhängigkeit von den Substanz-Sensordaten und/oder den davon abgeleitete Daten zumindest eine Maßnahme zu veranlassen. Beispielhafte Maßnahmen sind: das Veranlassen einer Nachbestellung einer in zumindest einem Behälter 120 gelagerten Substanz 121, und/oder das Senden von Information in Bezug auf die in den ein oder mehreren Behältern 120 gelagerten Substanzen 121 an ein elektronisches Gerät 130 (z.B. an ein Smartphone) eines Nutzers des Systems 100.

Eine Messvorrichtung 310 und/oder die Zentraleinheit 101 können eingerichtet sein, auf Basis der Vorrichtungsdaten (insbesondere der Bewegungsdaten) und/oder auf Basis der Substanz-Sensordaten zu bestimmen, ob ein Deckel 110 korrekt auf einen Behälter 120 aufgebracht wurde oder nicht. Mit anderen Worten, es kann detektiert werden, ob ein Behälter 120 korrekt mit einem Deckel 110 verschlossen wurde oder nicht. Die Zentraleinheit 101 kann eingerichtet sein, einen Hinweis an einen Nutzer (insbesondere an ein elektronisches Geräts 130 eines Nutzers) zu senden, wenn erkannt wird, dass ein Behälter 120 nicht korrekt mit einem Deckel 110 verschlossen wurde.

Die in diesem Dokument beschriebenen Maßnahmen ermöglichen es, die Haltbarkeit von Verbrauchsmaterialien zu erhöhen. Des Weiteren kann die Lagerhaltung von Verbrauchsmaterialien in einem Haushalt verbessert werden.

Die vorliegende Erfindung ist nicht auf die gezeigten Ausführungsbeispiele beschränkt. Insbesondere ist zu beachten, dass die Beschreibung und die Figuren nur das Prinzip des vorgeschlagenen Systems, der vorgeschlagenen Vorrichtung und/oder des vorgeschlagenen Verfahrens veranschaulichen sollen.

## Patentansprüche

1. Messvorrichtung (310) für einen Deckel (110) zum Abdecken eines Behälters (120), der zur Aufbewahrung einer Substanz (121) verwendet wird; wobei die Messvorrichtung (310) umfasst,
- zumindest einen Vorrichtungssensor (112, 118), der eingerichtet ist, Vorrichtungsinformation in Bezug auf eine Bewegung der Messvorrichtung (310) relativ zu dem Behälter (120) bereitzustellen;
- zumindest einen Inhaltssensor (114), der eingerichtet ist, Substanz-Sensordaten in Bezug auf die Substanz (121) in dem Behälter (120) zu erfassen;
- zumindest eine Kommunikationseinheit (117), die eingerichtet ist, Daten über ein Kommunikationsnetzwerk (102) zu versenden; und
- eine Steuereinheit (111), die eingerichtet ist,
- in Abhängigkeit von der Vorrichtungsinformation den Inhaltssensor (114) zu veranlassen, Substanz-Sensordaten zu erfassen; und
- die Kommunikationseinheit (117) zu veranlassen, die Substanz-Sensordaten und/oder davon abgeleitete Daten zu versenden;
**dadurch gekennzeichnet, dass**
- der Vorrichtungssensor (112, 118) einen Bewegungssensor (118) umfasst;
- die Vorrichtungsinformation Bewegungsdaten des Bewegungssensors (118) umfasst; und
- die Steuereinheit (111) eingerichtet ist, auf Basis der Bewegungsdaten zu bestimmen, ob der mit der Messvorrichtung (310) verbundene Deckel (110) auf den Behälter (120) aufgesetzt wurde oder von dem Behälter (120) abgenommen wurde.

2. Messvorrichtung (310) gemäß Anspruch 1, wobei
- die Messvorrichtung (310) ausgebildet ist, um an einem Deckel (110) lösbar befestigt zu werden; oder
- die Messvorrichtung (310) als fester Bestandteil in einen Deckel (110) integriert ist.

3. Messvorrichtung (310) gemäß einem der vorhergehenden Ansprüche, wobei
- der Inhaltssensor (114) einen Abstandssensor umfasst, der eingerichtet ist, Substanz-Sensordaten in Bezug auf einen Abstand (124) zwischen dem Abstandssensor und einer Oberfläche der Substanz (121) in dem Behälter (120) zu erfassen; und
- die Steuereinheit (111) eingerichtet ist, auf Basis der Substanz-Sensordaten einen Füllstand (122) der Substanz (121) in dem Behälter (120) zu ermitteln und/oder zu versenden.

4. Messvorrichtung (310) gemäß einem der vorhergehenden Ansprüche, wobei
- der Vorrichtungssensor (112, 118) einen Verschlusssensor (112) umfasst, der eingerichtet ist, zu detektieren, ob der mit der Messvorrichtung (310) verbundene Deckel (110) von dem Behälter (120) entfernt wird oder ist, oder ob der mit der Messvorrichtung (310) verbundene Deckel (110) von dem Behälter (120) auf den Behälter (120) aufgesetzt wird oder ist; und
- der Verschlusssensor (112) insbesondere einen Taster und/oder eine Lichtschranke umfasst.

5. Messvorrichtung (310) gemäß einem der vorhergehenden Ansprüche, wobei der Vorrichtungssensor (112, 118) eingerichtet ist, als Vorrichtungsinformation eine Energieversorgung der Steuereinheit (111) zu unterbinden oder eine Energieversorgung der Steuereinheit (111) nur dann zu ermöglichen, wenn der mit der Messvorrichtung (310) verbundene Deckel (110) von dem Behälter (120) entfernt wird oder ist, oder wenn der mit der Messvorrichtung (310) verbundene Deckel (110) auf den Behälter (120) aufgesetzt wird oder ist.

6. Messvorrichtung (310) gemäß einem der vorhergehenden Ansprüche, wobei die Steuereinheit (111) eingerichtet ist,
- auf Basis der Vorrichtungsinformation zu bestimmen, dass der mit der Messvorrichtung (310) verbundene Deckel (110) von dem Behälter (120) entfernt wird oder ist, oder dass der mit der Messvorrichtung (310) verbundene Deckel (110) auf den Behälter (120) aufgesetzt wird oder ist;
- in Reaktion darauf, den Inhaltssensor (114) zu veranlassen, Substanz-Sensordaten zu erfassen, und die Kommunikationseinheit (117) zu veranlassen, die Substanz-Sensordaten und/oder davon abgeleitete Daten zu versenden; und
- im Anschluss daran, die Messvorrichtung (310) in einen Schlaf-Zustand (224) mit reduziertem Energieverbrauch zu überführen.

7. Messvorrichtung (310) gemäß einem der vorhergehenden Ansprüche, wobei die Steuereinheit (111) eingerichtet ist,
- den Inhaltssensor (114) zu veranlassen, erste Substanz-Sensordaten mit einer reduzierten Genauigkeit in einem Energiespar-Modus zu erfassen;
- auf Basis der ersten Substanz-Sensordaten zu bestimmen, ob der mit der Messvorrichtung (310) verbundene Deckel (110) auf dem Behälter (120) aufgesetzt ist oder nicht; und
- den Inhaltssensor (114) zu veranlassen, Substanz-Sensordaten mit einer Standard-Genauigkeit in einem Standard-Modus zu erfassen, wenn oder sobald bestimmt wird, dass der mit der Messvorrichtung (310) verbundene Deckel (110) auf dem Behälter (120) aufgesetzt ist.

8. Messvorrichtung (310) gemäß einem der vorhergehenden Ansprüche, wobei
- die Messvorrichtung (310) ein oder mehrere Haltenasen (302) umfasst, die ausgebildet sind, durch einen Deckel (110) hindurch gesteckt zu werden, um die Messvorrichtung (310) an dem Deckel (110) zu befestigen; und/oder
- die Messvorrichtung (310) an einem Gehäuse ein Verbindungsmittel umfasst, das ausgebildet ist, mit einem komplementären Verbindungsmittel an einem Deckel (110) eine wiederholt lösbare Verbindung zu bilden, um die Messvorrichtung (310) an dem Deckel (110) zu befestigen.

9. Messvorrichtung (310) gemäß einem der vorhergehenden Ansprüche, wobei
- die Messvorrichtung (310) ausgebildet ist, von außen an einem Deckel (110) befestigt zu werden;
- der Inhaltssensor (114) ausgebildet ist, die Substanz-Sensordaten durch eine Öffnung (304) in dem Deckel (110) zu erfassen;
- die Messvorrichtung (310) insbesondere ein rohrförmiges Stanzwerkzeug (303) umfasst, das ausgebildet ist, eine Öffnung (304) in dem Deckel (110) für den Inhaltssensor (114) auszustanzen, wenn die Messvorrichtung (310) an dem Deckel (110) befestigt wird.

10. Messvorrichtung (310) gemäß einem der vorhergehenden Ansprüche, wobei
- der Bewegungssensor (118) einen Beschleunigungssensor und/oder eine Inertial Measurement Unit umfasst.

11. Messvorrichtung (310) gemäß einem der vorhergehenden Ansprüche, wobei die Messvorrichtung (310) umfasst,
- einen elektrischen Energiespeicher (115), der eingerichtet ist, elektrische Energie für den Betrieb der Messvorrichtung (310) zu speichern; und/oder
- eine Speichereinheit (116), die eingerichtet ist, zumindest vorübergehend Substanz-Sensordaten zu speichern.

12. Deckel (110) zum Abdecken eines Behälters (120); wobei der Deckel (110) eine Messvorrichtung (310) gemäß einem der vorhergehenden Ansprüche umfasst.

13. Deckel (110) gemäß Anspruch 12, wobei
- der Deckel (110) ein Gewinde (113) umfasst, das ausgebildet ist, in ein entsprechendes Gewinde eines Behälters (120) geschraubt zu werden; und
- der Vorrichtungssensor (112, 118) der Messvorrichtung (310) derart in oder an dem Gewinde (113) angeordnet ist, dass die Vorrichtungsinformation anzeigt, ob der Deckel (110) von dem Behälter (120) abgeschraubt oder auf den Behälter (120) aufgeschraubt wird.

14. System (100) zur Verwaltung des Inhalts von ein oder mehreren Behältern (120); wobei das System (100) umfasst,
- ein oder mehrere Messvorrichtungen (110) gemäß einem der Ansprüche 1 bis 11 wobei die ein oder mehrere Messvorrichtungen (110) ausgebildet sind, mit ein oder mehreren Deckeln (110) zum Abdecken der ein oder mehreren Behälter (120) verbunden zu werden, und/oder wobei die ein oder mehrere Messvorrichtungen (110) mit ein oder mehreren Deckeln (110) zum Abdecken der ein oder mehreren Behälter (120) verbunden sind; und
- eine Zentraleinheit (101), die eingerichtet ist,
- Substanz-Sensordaten und/oder davon abgeleitete Daten in Bezug auf den Inhalt der ein oder mehreren Behälter (120) von den ein oder mehreren Messvorrichtungen (310) zu empfangen; und
- in Abhängigkeit von den Substanz-Sensordaten und/oder den davon abgeleitete Daten zumindest eine Maßnahme zu veranlassen; wobei die Maßnahme insbesondere umfasst: Veranlassen einer Nachbestellung einer in zumindest einem Behälter (120) gelagerten Substanz (121), und/oder Senden von Information in Bezug auf die in den ein oder mehreren Behältern (120) gelagerten Substanzen (121) an ein elektronisches Gerät (130) eines Nutzers des Systems (100).

15. Verfahren (400) zur Bereitstellung von Information in Bezug auf eine Substanz (121), die in einem Behälter (120) aufbewahrt wird, mittels einer Messvorrichtung (310) für einen Deckel (110) zum Abdecken des Behälters (120); wobei die Messvorrichtung (310) zumindest einen Vorrichtungssensor (112, 118) mit einem Bewegungssensor (118) umfasst, der eingerichtet ist, Vorrichtungsinformation in Bezug auf eine Bewegung der Messvorrichtung (310) relativ zu dem Behälter (120) bereitzustellen; wobei die Messvorrichtung (310) zumindest einen Inhaltssensor (114) umfasst, der eingerichtet ist, Substanz-Sensordaten in Bezug auf die Substanz (121) in dem Behälter (120) zu erfassen; und wobei die Messvorrichtung (310) zumindest eine Kommunikationseinheit (117) umfasst, die eingerichtet ist, Daten über ein Kommunikationsnetzwerk (102) zu versenden; wobei das Verfahren (400) umfasst,
- Ermitteln (401) von Vorrichtungsinformation in Bezug auf eine Bewegung der Messvorrichtung (310) relativ zu dem Behälter (120), wobei die Vorrichtungsinformation Bewegungsdaten des Bewegungssensors (118) umfasst;
- Bestimmen auf Basis der Bewegungsdaten, ob der mit der Messvorrichtung (310) verbundene Deckel (110) auf den Behälter (120) aufgesetzt wurde oder von dem Behälter (120) abgenommen wurde;
- Erfassen (402) von Substanz-Sensordaten in Abhängigkeit von der Vorrichtungsinformation; und
- Versenden (403) der Substanz-Sensordaten und/oder davon abgeleiteter Daten.

## Claims

1. Measuring device (310) for a lid (110) for covering a container (120), which is used for the storage of a substance (121), wherein the measuring device (310) comprises:
- at least one device sensor (112, 118), which is designed to provide device information in respect of a movement of the measuring device (310) relative to the container (120);
- at least one contents sensor (114), which is designed to capture substance sensor data in respect of the substance (121) in the container (120);
- at least one communication unit (117), which is designed to send data via a communication network (102); and
- a control unit (111), which is designed
- to trigger the contents sensor (114) to capture substance sensor data as a function of the device information; and
- to trigger the communication unit (117) to send the substance sensor data and/or data derived therefrom;
**characterised in that**
- the device sensor (112, 118) comprises a movement sensor (118);
- the device information comprises movement data of the movement sensor (118); and
- the control unit (111) is designed to determine, on the basis of the movement data, whether the lid (110) connected to the measuring device (310) has been placed on the container (120) or removed from the container (120).

2. Measuring device (310) according to claim 1, wherein
- the measuring device (310) is embodied to be fastened detachably to a lid (110); or
- the measuring device (310) is integrated into a lid (110) as a fixed component.

3. Measuring device (310) according to one of the preceding claims, wherein
- the contents sensor (114) comprises a distance sensor, which is designed to capture substance sensor data in respect of a distance (124) between the distance sensor and a surface of the substance (121) in the container (120); and
- the control unit (111) is designed to determine and/or send a fill level (122) of the substance (121) in the container (120) on the basis of the substance sensor data.

4. Measuring device (310) according to one of the preceding claims, wherein
- the device sensor (112, 118) comprises a closing sensor (112), which is designed to detect whether the lid (110) connected to the measuring device (310) is being or has been removed from the container (120) or whether the lid (110) connected to the measuring device (310) is being or has been placed from the container (120) onto the container (120); and
- the closing sensor (112) comprises in particular a button and/or a light barrier.

5. Measuring device (310) according to one of the preceding claims, wherein the device sensor (112, 118) is designed to prevent, as device information, an energy supply to the control unit (111) or only to enable an energy supply to the control unit (111) if the lid (110) connected to the measuring device (310) is being or has been removed from the container (120) or if the lid (110) connected to the measuring device (310) is being or has been placed on the container (120).

6. Measuring device (310) according to one of the preceding claims, wherein the control unit (111) is designed
- to determine, on the basis of the device information, that the lid (110) connected to the measuring device (310) is being or has been removed from the container (120), or that the lid (110) connected to the measuring device (310) is being or has been placed on the container (120),
- in response, to trigger the contents sensor (114) to capture substance sensor data and to trigger the communication unit (117) to send the substance sensor data and/or data derived therefrom; and
- subsequently to transfer the measuring device (310) into a sleep state (224) with a reduced energy consumption.

7. Measuring device (310) according to one of the preceding claims, wherein the control unit (111) is designed
- to trigger the contents sensor (114) to capture first substance sensor data with a reduced accuracy in an energy saving mode,
- to determine on the basis of the first substance sensor data whether the lid (110) connected to the measuring device (310) has or has not been placed on the container (120); and
- to trigger the contents sensor (114) to capture substance sensor data with a standard accuracy in a standard mode if or as soon as it is determined that the lid (110) connected to the measuring device (310) has been placed on the container (120).

8. Measuring device (310) according to one of the preceding claims, wherein
- the measuring device (310) comprises one or more retaining lugs (302), which are embodied to be pushed through a lid (110) in order to fasten the measuring device (310) to the lid (110); and/or
- the measuring device (310) on a housing comprises a connecting means, which is embodied to form a repeatedly detachable connection with a complementary connecting means on a lid (110), in order to fasten the measuring device (310) to the lid (110).

9. Measuring device (310) according to one of the preceding claims, wherein
- the measuring device (310) is embodied to be fastened from the outside onto a lid (110);
- the contents sensor (114) is embodied to capture the substance sensor data through an opening (304) in the lid (110);
- the measuring device (310) comprises in particular a tubular punching tool (303), which is embodied to punch an opening (304) in the lid (110) for the contents sensor (114) if the measuring device (310) is fastened to the lid (110).

10. Measuring device (310) according to one of the preceding claims, wherein
- the movement sensor (118) comprises an acceleration sensor and/or an inertial measuring unit.

11. Measuring device (310) according to one of the preceding claims, wherein the measuring device (310) comprises
- an electrical energy store (115), which is designed to store electrical energy for the operation of the measuring device (310); and/or
- a storage unit (116), which is designed to store substance sensor data at least temporarily.

12. Lid (110) for covering a container (120), wherein the lid (110) comprises a measuring device (31) according to one of the preceding claims.

13. Lid (110) according to claim 12, wherein
- the lid (110) comprises a thread (113), which is embodied to be screwed into a corresponding thread of a container (120); and
- the device sensor (112, 118) of the measuring device (310) is arranged in or on the thread (113) so that the device information shows whether the lid (110) is being unscrewed from the container (120) or screwed onto the container (120).

14. System (100) for managing the contents of one or more containers (120), wherein the system (100) comprises
- one or more measuring devices (110) according to one of claims 1 to 11, wherein the one or more measuring devices (110) are embodied to be connected to one or more lids (110) for covering the one or more containers (120), and/or wherein the one or more measuring devices (110) are connected to one or more lids (110) for covering the one or more containers (120); and
- a central unit (101), which is designed
- to receive substance sensor data and/or data derived therefrom in respect of the contents of the one or more containers (120) from the one or more measuring devices (310); and
- to trigger at least one measure as a function of the substance sensor data and/or the data derived therefrom, wherein the measure in particular comprises: triggering a reorder of a substance (121) stored in at least one container (120), and/or sending information in respect of the substances (121) stored in the one or more containers (120) to an electronic device (130) of a user of the system (100).

15. Method (400) for providing information in respect of a substance (121), which is stored in a container (120), by means of a measuring device (310) for a lid (110) for covering the container (120), wherein the measuring device (310) comprises at least one device sensor (112, 118) with a movement sensor (118), which is designed to provide device information in respect of a movement of the measuring device (310) relative to the container (120), wherein the measuring device (310) comprises at least one contents sensor (114), which is designed to capture substance sensor data in respect of the substance (121) in the container (120), and wherein the measuring device (310) comprises at least one communication unit (117), which is designed to send data via a communication network (102), wherein the method (400) comprises
- determining (401) device information in respect of a movement of the measuring device (310) relative to the container (120), wherein the device information comprises movement data of the movement sensor (118);
- determining on the basis of the movement data whether the lid (110) connected to the measuring device (310) has been placed on the container (120) or removed from the container (120);
- capturing (402) substance sensor data as a function of the device information; and
- sending (403) substance sensor data and/or data derived therefrom.

## Revendications

1. Dispositif de mesure (310) pour un couvercle (110) destiné à recouvrir un récipient (120), qui est utilisé pour conserver une substance (121), dans lequel le dispositif de mesure (310) comprend :
- au moins un capteur de dispositif (112, 118), qui est adapté pour fournir des informations de dispositif concernant un déplacement du dispositif de mesure (310) par rapport au récipient (120),
- au moins un capteur de contenu (114), qui est adapté pour recueillir des données de détection de substance concernant la substance (121) dans le récipient (120),
- au moins une unité de communication (117), qui est adaptée pour envoyer des données via un réseau de communications (102), et
- une unité de commande (111), qui est adaptée pour :
- en fonction des informations de dispositif, inciter le capteur de contenu (114) à recueillir des données de détection de substance, et
- inciter l'unité de communication (117) à envoyer les données de détection de substance et/ou des données dérivées de celles-ci,
**caractérisé en ce que** :
- le capteur de dispositif (112, 118) comprend un capteur de mouvement (118),
- les informations de dispositif comprennent des données de déplacement du capteur de mouvement (118), et
- l'unité de commande (111) est configurée pour déterminer, sur la base des données de déplacement, si le couvercle (110) relié au dispositif de mesure (310) a été positionné sur le récipient (120) ou s'il a été enlevé du récipient (120).

2. Dispositif de mesure (310) selon la revendication 1, dans lequel :
- le dispositif de mesure (310) est configuré pour être fixé de façon détachable à un couvercle (110), ou
- le dispositif de mesure (310) est intégré sous forme d'un composant fixe dans un couvercle (110).

3. Dispositif de mesure (310) selon l'une des revendications précédentes, dans lequel
- le capteur de contenu (114) comprend un capteur de distance, qui est adapté pour recueillir des données de détection de substance concernant une distance (124) entre le capteur de distance et une surface de la substance (121) dans le récipient (120), et
- l'unité de commande (111) est configurée pour déterminer et/ou envoyer un niveau de remplissage (122) de la substance (121) dans le récipient (120) sur la base des données de détection de substance.

4. Dispositif de mesure (310) selon l'une des revendications précédentes, dans lequel
- le capteur de dispositif (112, 118) comprend un capteur de fermeture (112) qui est adapté pour détecter si le couvercle (110) relié au dispositif de mesure (310) est en train d'être ou est enlevé du récipient (120) ou si le couvercle (110) relié au dispositif de mesure (310) est en train d'être ou est positionné sur le récipient (120), et
- le capteur de fermeture (112) comprend en particulier un palpeur et/ou une barrière lumineuse.

5. Dispositif de mesure (310) selon l'une des revendications précédentes, dans lequel le capteur de dispositif (112, 118) est adapté pour interdire, à titre d'informations de dispositif, une alimentation d'énergie de l'unité de commande (111), ou permettre une alimentation d'énergie de l'unité de commande (111) seulement lorsque le couvercle (110) relié au dispositif de mesure (310) est en train d'être ou est enlevé du récipient (120), ou lorsque le couvercle (110) relié au dispositif de mesure (310) est en train d'être ou est positionné sur le récipient (120).

6. Dispositif de mesure (310) selon l'une des revendications précédentes, dans lequel l'unité de commande (111) est adaptée pour
- déterminer, sur la base des informations de dispositif, que le couvercle (110) relié au dispositif de mesure (310) est en train d'être ou est enlevé du récipient (120), ou que le couvercle relié au dispositif de mesure (310) est en train d'être ou est positionné sur le récipient (120),
- en réaction à cela, inciter le capteur de contenu (114) à recueillir des données de détection de substance et inciter l'unité de communication (117) à envoyer les données de détection de substance et/ou des données dérivées de celles-ci, et
- à la suite de cela, transférer le dispositif de mesure (310) dans un état de sommeil (224) ayant une consommation d'énergie réduite.

7. Dispositif de mesure (310) selon l'une des revendications précédentes, dans lequel l'unité de commande (111) est adaptée pour
- inciter le capteur de contenu (114) à recueillir des premières données de détection de substance avec une précision réduite dans un mode d'économie d'énergie,
- déterminer, sur la base des premières données de détection de substance, si le couvercle (110) relié au dispositif de mesure (310) est positionné ou non sur le récipient (120), et
- inciter le capteur de contenu (114) à recueillir des données de détection de substance avec une précision standard dans un mode standard, lorsqu'il ou dès qu'il est déterminé que le couvercle (110) relié au dispositif de mesure (310) est positionné sur le récipient (120).

8. Dispositif de mesure (310) selon l'une des revendications précédentes, dans lequel
- le dispositif de mesure (310) comprend un ou plusieurs becs de retenue (302), qui sont configurés pour être insérés à travers un couvercle (110) afin de fixer le dispositif de mesure (310) sur le couvercle (110), et/ou
- le dispositif de mesure (310) comprend un moyen de liaison sur un boîtier, qui est configuré pour former une liaison détachable à maintes reprises avec un moyen de liaison complémentaire sur un couvercle (110), afin de fixer le dispositif de mesure (310) sur le couvercle (110).

9. Dispositif de mesure (310) selon l'une des revendications précédentes, dans lequel
- le dispositif de mesure (310) est configuré pour être fixé de l'extérieur sur un couvercle (110),
- le capteur de contenu (114) est configuré pour recueillir les données de détection de substance à travers une ouverture (304) dans le couvercle (110),
- le dispositif de mesure (310) comprend en particulier un outil de poinçonnage (303) tubulaire, qui est configuré pour découper par poinçonnage une ouverture (304) dans le couvercle (110) pour le capteur de contenu (114), lorsque le dispositif de mesure (310) est fixé au couvercle (110).

10. Dispositif de mesure (310) selon l'une des revendications précédentes, dans lequel
- le capteur de mouvement (118) comprend un capteur d'accélération et/ou un dispositif de mesure inertielle.

11. Dispositif de mesure (310) selon l'une des revendications précédentes, dans lequel le dispositif de mesure (310) comprend :
- un accumulateur d'énergie électrique (115), qui est adapté pour emmagasiner de l'énergie électrique pour le fonctionnement du dispositif de mesure (310), et/ou
- une unité de mémoire (116), qui est adaptée pour stocker au moins temporairement des données de détection de substance.

12. Couvercle (110) pour recouvrir un récipient (120), dans lequel le couvercle (110) comprend un dispositif de mesure (310) selon l'une des revendications précédentes.

13. Couvercle (110) selon la revendication 12, dans lequel
- le couvercle (110) comprend un filetage (113), qui est configuré pour être vissé dans un filetage correspondant d'un récipient (120), et
- le capteur de dispositif (112, 118) du dispositif de mesure (310) est agencé dans ou sur le filetage (113) de telle manière que les informations de dispositif indiquent si le couvercle (110) est dévissé du récipient (120) ou s'il est vissé sur le récipient (120).

14. Système (100) de gestion d'un contenu d'un ou plusieurs récipients (120), dans lequel le système (100) comprend :
- un ou plusieurs dispositifs de mesure (310) selon l'une des revendications 1 à 11, dans lequel l'un ou plusieurs dispositifs de mesure (310) sont configurés pour être reliés à un ou plusieurs couvercles (110) pour recouvrir l'un ou plusieurs récipients (120), et/ou dans lequel l'un ou plusieurs dispositifs de mesure (310) sont reliés à l'un ou plusieurs couvercles (110) pour recouvrir l'un ou plusieurs récipients (120), et
- une unité centrale (101), qui est configurée pour
- recevoir des données de détection de substance et/ou des données dérivées de celles-ci concernant le contenu d'un ou plusieurs récipients (120) de l'un ou plusieurs dispositifs de mesure (310), et
- en fonction des données de détection de substance et/ou des données dérivées de celles-ci, initier au moins une mesure, dans lequel la mesure comprend notamment : l'initiation d'une commande de réapprovisionnement d'une substance (121) stockée dans au moins un récipient (120) et/ou l'envoi d'informations concernant les substances (121) stockées dans l'un ou plusieurs récipients (120) à un appareil électronique (130) d'un utilisateur du système (100).

15. Procédé (400) de fourniture d'informations concernant une substance (121), qui est conservée dans un récipient (120), au moyen d'un dispositif de mesure (310) pour un couvercle (110) destiné à recouvrir le récipient (120),
dans lequel le dispositif de mesure (310) comprend un capteur de dispositif (112, 118) incluant un capteur de mouvement (118), qui est adapté pour fournir des informations de dispositif concernant un déplacement du dispositif de mesure (310) par rapport au récipient (120),
dans lequel le dispositif de mesure (310) comprend au moins un capteur de contenu (114), qui est adapté pour recueillir des données de détection de substance concernant la substance (121) dans le récipient (120), et
dans lequel le dispositif de mesure (310) comprend au moins une unité de communication (117), qui est adaptée pour envoyer des données via un réseau de communications (102),
dans lequel le procédé (400) comprend :
- la détermination (401) d'informations de dispositif concernant un déplacement du dispositif de mesure (310) par rapport au récipient (120), dans lequel les informations de dispositif sont des données de déplacement du capteur de mouvement (118),
- la détermination, sur la base des données de déplacement, si le couvercle (110) relié au dispositif de mesure (310) a été positionné sur le récipient (120) ou s'il a été retiré du récipient (120),
- le recueil (402) de données de détection de substance en fonction des informations de dispositif, et
- l'envoi (403) des données de détection de substance et/ou de données dérivées de celles-ci.
